(19)

Europäisches
Patentamt
European
Patent Office
Office européen
des brevets

(11) **EP 2 380 955 A1**

(12) **EUROPEAN PATENT APPLICATION**
published in accordance with Art. 153(4) EPC

(43) Date of publication:
**26.10.2011 Bulletin 2011/43**

(21) Application number: **09834659.6**

(22) Date of filing: **18.11.2009**

(51) Int Cl.:
*C11D 1/28* (2006.01)     *A61K 8/46* (2006.01)
*A61Q 5/02* (2006.01)     *A61Q 19/10* (2006.01)
*C07C 309/17* (2006.01)     *C11D 11/04* (2006.01)
*C07B 61/00* (2006.01)

(86) International application number:
**PCT/JP2009/069554**

(87) International publication number:
**WO 2010/073855 (01.07.2010 Gazette 2010/26)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **22.12.2008 JP 2008326217**
**26.12.2008 JP 2008332748**

(71) Applicant: **Lion Corporation**
**Tokyo 130-8644 (JP)**

(72) Inventors:
• **KONTA, Hiroshi**
**Tokyo 130-8644 (JP)**

• **NOMURA, Masako**
**Tokyo 130-8644 (JP)**
• **OBAYASHI, Yuko**
**Tokyo 130-8644 (JP)**

(74) Representative: **Hill, Christopher Michael**
**Page White & Farrer**
**Bedford House**
**John Street**
**London, WC1N 2BF (GB)**

(54) **CLEANSER COMPOSITION, PROCESS FOR PRODUCING SAME, AMPHIPHILIC COMPOUND, AND COMPOSITION**

(57) To provide a detergent composition, containing: a compound expressed by the following general formula (1):

$$C_{10}\text{-CH(SO}_3\text{A)-CONR'R''} \qquad \text{General Formula (1)}$$

where $C_{10}$ is a C10 linear or branched alkyl chain; R' is a hydrogen atom or a C1-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group.

**EP 2 380 955 A1**

## Description

[Technical Field]

The present invention relates to a detergent composition containing an alkanolamide compound of α-sulfo fatty acid and production method thereof, as well as relating to an amphiphilic compound consisted of an alkanolamide compound of α-sulfo fatty acid, a composition containing such amphiphilic compound, a detergent composition containing such composition, and washing method using the same.

[Background Art]

Alpha-alkyl ester sulfonates, particularly alpha-methyl ester sulfonates (MES), can be produced from vegitable oils, and therefore are compounds that are expected to be used in the near future as environmentally friendly surfactants. As needs of customers for surfactants, there are demands for activating agents that give mild effects on skins or hair of humans, which is reflected from the fact that numbers of users having sensitive skins have increased.
Therefore, it has been desired that a milder activating agent, which can be derived from environmentally friendly alpha-alkyl ester sulfonates and reduces adverse effects such as protein denaturation or skin irritation, be developed.
Various alpha-alkyl ester sulfonates and derivatives thereof have been known (see NPLs 1 to 3, and PTL 1). NPLs 1 and 2 disclose $C_{16}$-$C_{18}$ α-sulfo fatty acid amide compounds (may also referred to as SF amide hereinafter) having a specific molecular structure, which is produced from α-sulfo fatty acid chloride, and discuss dissolution temperature, stability in hard water, and scum diffusion effect as basic physical properties of the SF amide.
However, in NPLs 1 and 2, there are no descriptions related to mildness in connection with protein denaturation, and skin irritation, and thus there is still problem that an activating agent having improved mildness has not yet be proposed.
Moreover, NPL 3 discloses a $C_{16}$ to $C_{18}$ α-sulfo fatty acid alkanol amide compound (may also be referred to as SF amide alcohol hereinafter) having a specific molecular structure, which is synthesized from a benzene solvent containing MES as a starting material, and discuss dissolution temperature, stability in hard water, scum diffusion, and cleansing power on a stained cloth with a soap mixture, as basic physical properties and laundry cleansing power of the SF amide alcohol.
However, in NPL 3, there are no descriptions related to mildness in connection with protein denaturation, and skin irritation, and thus there is still problem that an activating agent having improved mildness has not yet be proposed.
Moreover, although there is an evaluation of the SF amide alcohol on the cleansing powder to the stained cloth as a detergent composition in NPL 3, there are no descriptions or suggestions for applications thereof as a body washing or house cleaning detergent.
Furthermore, PTL 1 discloses a two-chains and two-hydrophilic groups type of an α-sulfo fatty acid amide compound. It is disclosed in PTL 1 that the two-chains and two-hydrophilic groups type of the α-sulfo fatty acid amide compound has, as a detergent composition, excellent resistance in hard water, excellent dispersibility, high effect for preventing reattachment of stain, and low skin irritation.
However, the further improvement is still desired for reducing protein denaturation, as it is desired that the detergent or the like of such compound be used more safely for users including users having sensitive skins.
Moreover, when alpha-alkyl ester sulfonates are applied for a liquid detergent, they often cause hydrolysis. Therefore, there has been a demand for a detergent composition that can be stably applied for a detergent.

[Citation List]

[Patent Literature]

[PTL1] Japanese Patent Application Laid-Open (JP-A) No. 06-330084

[Non Patent Literature]

[NPL1] J. Am. Oil Chem. Soc., 1960, 37, 295-297
[NPL2] J. Am. Oil Chem. Soc., 1962, 39, 490-496
[NPL3] J. Am. Oil Chem. Soc., 1974, 51, 435-438

[Summary of Invention]

[Technical Problem]

The present invention aims at solving various problems in the art, and achieving the following object. Specifically, an object of the present invention is to provide a milder detergent composition with reduced protein denaturation or skin irritation, and a production method thereof.

[Solution to Problem]

The means for solving the aforementioned problems are as follows:

<1> A detergent composition, containing:

a compound expressed by the following general formula (1):

$$C_{10}\text{-CH(SO}_3\text{A)-CUNR'R''} \qquad \text{General Formula (1)}$$

where $C_{10}$ is a C10 linear or branched alkyl chain; R' is a hydrogen atom or a C1-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group.
<2> A detergent composition, containing:

a compound expressed by the following general formula (2):

$$C_{12}\text{-CH(SO}_3\text{A)-CONR'R''} \qquad \text{General Formula (2)}$$

where $C_{12}$ is a C12 linear or branched alkyl chain; R' is a hydrogen atom or a C1-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group.
<3> A detergent composition, containing:

a compound expressed by the following general formula (3):

$$C_{14}\text{-CH(SO}_3\text{A)-CONR'R''} \qquad \text{General Formula (3)}$$

where $C_{14}$ is a C14 linear or branched alkyl chain; R' is a hydrogen atom or a C1-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group,
wherein the detergent composition is for body washing, dish washing, bathroom cleaning, or house cleaning.
<4> A detergent composition, containing:

a compound expressed by the following general formula (4):

$$C_{16}\text{-CH(SO}_3\text{A)-CONR'R''} \qquad \text{General Formula (4)}$$

where $C_{16}$ is a C16 linear or branched alkyl chain; R' is a hydrogen atom or a C1-3 alkyl group which may contain a hydroxy group R'' is a C2-6 alkyl group which contains one hydroxy group; and A is an alkali metal, an alkaline earth metal, or C6 or smaller protonated amine which may contain a hydroxy group,
wherein the detergent composition is for body washing, dish washing, bathroom cleaning, or house cleaning.

<5> A method for producing a detergent composition, containing:

heating an alcohol solvent containing an α-sulfo fatty acid alkyl ester salt expressed by the following general formula (5), alkanolamine, and metal alkoxide to produce the detergent composition as defined in any one of <1> to <4>:

**R-CH(SO₃A)COOR'''**       General Formula (5)

where R is a C10, 12, 14, or 16 linear or branched alkyl chain; R''' is a C1-4 linear or branched alkyl chain; and A is an alkali metal, an alkaline earth metal, or C6 or smaller protonated amine which may contain a hydroxy group.

<6> An amphiphilic compound, having a molecular structure expressed by the following general formula (I):

**R-CH (SO₃A)-CONHCH₂CH(OH)CH₂OH**       General Formula (I)

where R is a C 8-16 linear or branched alkyl chain; and A is an alkali metal, an alkaline earth metal, or C6 or smaller protonated amine which may contain a hydroxy group.

<7> A composition, containing the amphiphilic compound as defined in <6>.

<8> A detergent composition, containing the amphiphilic compound as defined in <6>.

<9> A method for producing a detergent composition, containing:

heating an alcohol solvent containing an α-sulfo fatty acid alkyl ester salt expressed by the following general formula (II), 3-amino-1,2-propanediol, and metal alkoxide to produce the detergent composition as defined in <8>:

**R-CH(SO₃A)COOR'**       General Formula (II)

where R is a C8-16 linear or branched alkyl chain; R' is a C1-4 linear or branched alkyl chain; and A is an alkali metal, an alkaline earth metal, or C6 or smaller protonated amine which may contain a hydroxy group.

[Advantageous Effects of Invention]

The present invention solves various problems in the art and achieves the aforementioned object, and moreover can provide a milder detergent composition with reduced protein denaturation or skin irritation, and a production method thereof.

[Description of Embodiments]

(Detergent Composition)

The detergent composition of the present invention contains an alkanolamide compound of α-sulfo fatty acid, particulary an alkanolamide compound of α-sulfo lauric acid (C₁₂SF amide alcohol), an alkanolamide compound of α-sulfo myristic acid (C₁₄SF amide alcohol), an alkanolamide compound of α-sulfo palmitic acid (C₁₆SF amide alcohol), or an alkanolamide compound of α-sulfo stearic acid (C₁₈SF amide alcohol), all of which will be explained later.

-C₁₂SF Amide Alcohol-

The C₁₂SF amide alcohol is a compound expressed by the following general formula (1).

**C₁₀-CH (SO₃A)-CONR'R''**       General Formula (1)

In the general formula (1), C₁₀ is a C10 linear or branched alkyl chain; R' is a hydrogen atom or a C1-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group.

The alkali metal is not particularly restricted, but it is preferably selected from lithium, sodium, and potassium in view of its availability and cost.

Moreover, the alkaline earth metal is not particularly restricted, but it is preferably selected from magnesium, and calcium in view of its availability and cost.

The C₁₂SF amide alcohol is suitably selected depending on the intended purpose without any restriction, provided that it is a compound expressed by the general formula (1), but it is preferably selected from a monoethanol amide compound, an isopropanol amide compound, a 2-(2-hydroxyethoxy)ethyl amide compound, and a N-methyl-N-2-hydroxyethyl amide compound in view of its availability and cost.

As characteristics of the $C_{12}SF$ amide alcohol, the $C_{12}SF$ amide alcohol has low protein denaturation, and excellent stability in hard water, as well as having sufficient foamability, detergency and water-solubility, and hence the $C_{12}SF$ amide alcohol is suitably used for body washing, dish washing, bathroom cleaning, house cleaning, and laundry washing.

-$C_{14}SF$ Amide Alcohol-

The $C_{14}SF$ amide alcohol is a compound expressed by the following general formula (2).

$$C_{12}\text{-CH (SO}_3\text{A)-CONR'R''} \qquad \text{General Formula (2)}$$

In the general formula (2), $C_{12}$ is a C12 linear or branched alkyl chain; R' is a hydrogen atom or a C1-3 alkyl group which may contain a hydroxy group; R" is a C2-6 alkyl group which contains one hydroxy group; and A is an alkali metal, an alkaline earth metal, or C6 or smaller protonated amine which may contain a hydroxy group.

The alkali metal is not particularly restricted, but it is preferably selected from lithium, sodium, and potassium in view of availability of a raw material thereof and its cost.
Moreover, the alkaline earth metal is not particularly restricted, but it is preferably selected from magnesium, and calcium in view of its availability and cost.

The $C_{14}SF$ amide alcohol is suitably selected depending on the intended purpose without any restriction, provided that it is a compound expressed by the general formula (2), but it is preferably selected from a monoethanol amide compound, an isopropanol amide compound, a 2-(2-hydroxyethoxy)ethyl amide compound, and a N-methyl-N-2-hydroxyethyl amide compound in view of its availability and cost.

As characteristics of the $C_{14}SF$ amide alcohol, the $C_{14}SF$ amide alcohol has low protein denaturation, and excellent stability in hard water, as well as having sufficient foamability, detergency and water-solubility, and hence the $C_{14}SF$ amide alcohol is suitably used for body washing, dish washing, bathroom cleaning, house cleaning, and laundry washing.

- $C_{16}SF$ Amide Alcohol-

The $C_{16}SF$ amide alcohol is a compound expressed by the following general formula (3).

$$C_{14}\text{-CH (SO}_3\text{A)-CONR'R''} \qquad \text{General Formula (3)}$$

In the general formula (3), $C_{14}$ is a C14 linear or branched alkyl chain; R' is a hydrogen atom or a C1-3 alkyl group which may contain a hydroxy group; R" is a C2-6 alkyl group which contains one hydroxy group; and A is an alkali metal, an alkaline earth metal, or C6 or smaller protonated amine which may contain a hydroxy group.

The alkali metal is not particularly restricted, but it is preferably selected from lithium, sodium, and potassium in view of its availability and cost.
Moreover, the alkaline earth metal is not particularly restricted, but it is preferably selected from magnesium, and calcium in view of its availability and cost.

The $C_{16}SF$ amide alcohol is suitably selected depending on the intended purpose without any restriction, provided that it is a compound expressed by the general formula (3), but it is preferably selected from a monoethanol amide compound, an isopropanol amide compound, a 2-(2-hydroxyethoxy)ethyl amide compound, and a N-methyl-N-2-hydroxyethyl amide compound in view of its availability and cost.

As characteristics of the $C_{16}SF$ amide alcohol, the $C_{16}SF$ amide alcohol has low protein denaturation, as well as having sufficient foamability, and detergency.
Therefore, the detergent composition containing the $C_{16}SF$ amide alcohol is suitably used for body washing, dish washing, bathroom cleaning, and house cleaning.

-$C_{18}SF$ Amide Alcohol-

The $C_{18}SF$ amide alcohol is a compound expressed by the following general formula (4).

$$C_{16}\text{-CH (SO}_3\text{A)-CONR'R''} \qquad \text{General Formula (4)}$$

In the general formula (4), $C_{16}$ is a C16 linear or branched alkyl chain; R' is a hydrogen atom or a C1-3 alkyl group which may contain a hydroxy group; R" is a C2-6 alkyl group which contains one hydroxy group; and A is an alkali metal, an alkaline earth metal, or C6 or smaller protonated amine which may contain a hydroxy group.

The alkali metal is not particularly restricted, but it is preferably selected from lithium, sodium, and potassium in view of its availability and cost.

Moreover, the alkaline earth metal is not particularly restricted, but it is preferably selected from magnesium, and calcium in view of its availability and cost.

The $C_{18}$SF amide alcohol is suitably selected depending on the intended purpose without any restriction, provided that it is a compound expressed by the general formula (4), but it is preferably selected from a monoethanol amide compound, an isopropanol amide compound, a 2-(2-hydroxyethoxy)ethyl amide compound, and a N-methyl-N-2-hydroxyethyl amide compound in view of its availability and cost.

The characteristics of the $C_{18}$SF amide alcohol include low protein denaturation ability, and sufficient detergency.

The detergent composition containing the $C_{18}$SF amide alcohol is used for body washing, dish washing, bathroom cleaning and house cleaning.

The detergent containing any of the $C_{12}$SF amide alcohol, the $C_{14}$SF amide alcohol, the $C_{16}$SF amide alcohol, or the $C_{18}$SF amide alcohol may contain one SF amide alcohol independently, or two or more SF amide alcohols in combination.

An amount of the detergent composition contained in a detergent is suitably selected depending on the intended purpose without any restriction, but it is preferably 100% by mass to 0.1% by mass.

The detergent composition exhibits sufficient detergency with the activator concentration of 0.01% by mass. For example, the detergent composition can be used as a house cleaning detergent. Moreover, the detergent composition can be used as a soap, such as a bar soap (100% by mass).

A pH of the detergent containing the detergent composition is suitably selected depending on the intended purpose without any restriction, but it is preferably 4 to 12.

Since the SF amide alcohol has an amide bond, it does not easily cause hydrolysis, which esters often causes, and thus it is stable in a wide pH range.

Accordingly, in the case where the detergent composition is applied for a detergent, it can be widely applied, for example as a detergent for body washing, for a subacidic detergent (having pH of about 5 to about 7) as well as a soap which is alkaline. Moreover, the detergent composition is widely applied, for example, for a relatively strong alkaline detergent such as of pH of about 10, as a detergent for house cleaning.

For the purpose of controlling the pH, a pH regulator may be used in detergent composition.

The pH regulator is not particularly restricted, but it is preferably citric acid, sodium hydroxide, p-toluene sulfonic acid, or the like in view of its availability and cost.

-Other Components-

Other components contained in the detergent containing the detergent composition are suitably selected depending on the intended purpose without any restriction. For example, conventional components formulated into a detergent, such as fragrance materials, other active agents, solvents, and the like can be formulated in accordance with the conventional formulations known in the art.

For example, a builder such as zeolite, sodium(potassium) carbonate, sodium silicate, sodium citrate, and polyacrylate; a moisturizing agent such as propylene glycol, glycerin, and sorbitol; a viscosity modifier such as methyl cellulose, polyoxyethylene glycol distearate, and ethanol; an antiseptic agent such as methylparaben, and butylparaben; an anti-inflammatory agent such as potassium glycyrrhizinate, and tocopheryl acetate; others such as a sterilizer, a pearling agent, an antioxidant, a fragrance material, a colorant, and an ultraviolet absorber can be formulated, if necessary.

It is also possible that another surfactant is used in combination in the detergent. Preferable examples of such surfactant include: an anionic surfactant such as alkylbenzene sulfonate, α-olefin sulfonate, α-sulfo fatty acid alkyl ester salts, alkyl sulfuric acid ester salts, polyoxyethylene alkyl sulfuric acid ester salts, alkyl phosphoric acid ester-based surfactants, amino acid-based surfactants, sulfo-succinic acid-based surfactants, taurate-based surfactants, and higher fatty acid salts; and a nonionic surfactant such as alkyl saccharide-based surfactants, and polyoxyethylene alkyl ether-based surfactant.

(Method for Producing Detergent Composition)

The method for producing a detergent composition of the present invention contains heating an alcohol solvent containing the α-sulfo fatty acid alkyl ester salt, alkanolamine, and metal alkoxide to produce the detergent composition.

The detergent composition can also be produced by using a solvent other than alcohol, but according to the method for producing the detergent composition of the present invention, which uses the alcohol solvent, use of solvents such as benzene that has carcinogenicity, and toluene that is toxic, can be avoided and thus a production process having low loads to the environment can be realized.

The α-sulfo fatty acid alkyl ester salt is a compound expressed by the following general formula (5):

$$R\text{-}CH(SO_3A)COOR'''$$ General Formula (5)

In the general formula (5), R is a C10, 12, 14, or 16 linear or branched alkyl chain; R''' is a C1-4 linear or branched alkyl chain; and A is an alkali metal, an alkaline earth metal, or C6 or smaller protonated amine which may contain a hydroxy group.

The α-sulfo fatty acid alkyl ester salt is appropriately selected depending on the length of the alkyl chain of the alkanolamide compound of the α-sulfo fatty acid alkyl ester salt to be produced.

For example, in the case where the $C_{12}$SF amide alcohol is produced, an α-sulfo lauric acid alkyl ester salt can be used; in the case where the $C_{14}$SF amide alcohol is produced, an α-sulfo myristic acid alkyl ester salt can be used; in the case where the $C_{16}$SF amide alcohol is produced, an α-sulfo palmitic acid alkyl ester salt can be used; and in the case where the $C_{18}$SF amide alcohol is produced, an α-sulfo stearic acid alkyl ester salt can be used.

Moreover, a number of carbon atoms of the alkyl ester in the α-sulfo fatty acid alkyl ester salt is not particularly restricted, but the number of the carbon atoms thereof is preferably 1 to 4 in view of the production efficiency.

The salt is not particularly restricted, but it is preferably lithium, sodium, potassium, magnesium, or calcium, in view of its availability and cost.

The alkanolamine is appropriately selected depending on the SF amide alcohol to be produced without any restriction.

For example, in the case where a monoethanol amide compound is produced, 2-aminoethanol can be used; in the case where an isopropanol amide compound is produced, 3-amino-2-propanol can be used; in the case where a 2-(2-hydroxyethoxy)ethyl amide compound is produced, 2-(2-aminoethoxy)ethanol can be used; and in the case where an N-methyl-N-2-hydroxyethyl amide compound is produced, N-methyl-N-2-hydroxyethyl amine can be used.

An amount of the alkanolamine to be formulated is not particularly restricted, but it is preferably 0.5 Eq to 1.5 Eq relative to 1 Eq of the α-sulfo fatty acid alkyl ester salt.

When the amount thereof is less than 0.5 Eq, a large amount of the α-sulfo fatty acid alkyl ester salt is remained without being reacted, requiring a complicated purification process. When the amount thereof is more than 1.5 Eq, the cost increases by the excess amount of the alkanolamine, and therefore it is not preferable.

The alcohol solvent is not particularly restricted, and examples thereof include alcohols such as methanol, ethanol, propanol, and butanol.

An amount of the alcohol solvent for use is not particularly restricted, but it is preferably 1 part by mass to 10 parts by mass relative to 1 part by mass of the α-sulfo fatty acid alkyl ester salt.

When the amount thereof is less than 1 part by mass, a reactant is not dissolved, which makes stirring difficult. When the amount thereof is more than 10 parts by mass, a concentration of the resulting reaction product is low, and the productivity thereof is poor, and thus it is not preferable.

The temperature for heating the alcohol solvent containing the α-sulfo fatty acid alkyl ester salt, the alkanolamine, and the metal alkoxide is not particularly restricted, but it is preferably 60°C to 170°C.

When the temperature for heating is lower than 60°C, the reaction speed is slow, which causes reduction in the production efficiency. When the temperature for heating is higher than 170°C, part of the reaction product is decomposed, and tinted, and therefore it is not preferable.

The metal alkoxide is not particularly restricted, but it is preferably selected from metal alkoxides such as methanol, ethanol, propanol, or butanol of lithium, potassium, sodium, or magnesium in view of its availability and reactivity.

An amount of the metal alkoxide for use is not particularly restricted, but it is preferably 0.03 Eq to 2 Eq relative to 1 Eq of the α-sulfo fatty acid alkyl ester salt.

When the amount thereof is less than 0.03 Eq, the reaction speed is very slow, which causes reduction in the production efficiency. When the amount thereof is more than 2 Eq, the cost increases, and thus it is not preferable.

(Amphiphilic Compound)

The amphiphilic compound of the present invention is a compound expressed by the following general formula (I) (i.e. a 2,3-dihydroxypropyl amide compound of α-sulfo fatty acid):

$$R\text{-}CH(SO_3A)\text{-}CONHCH_2CH(OH)CH_2OH$$ General Formula (I)

In the general formula (I), R is a C 8-16 linear or branched alkyl chain; and A is an alkali metal, an alkaline earth metal, or C6 or smaller protonated amine which may contain a hydroxy group.

"R" is not particularly restricted as long as it is a C8-16 linear or branched alkyl chain, and it is preferably C10-16 linear or branched alkyl chain in view of the resulting cleansing ability and availability of a starting fatty acid compound.

The alkali metal is not particularly restricted, but it is preferably selected from lithium, sodium, and potassium in view of its availability and cost.

Moreover, the alkaline earth metal is not particularly restricted, but it is preferably selected from magnesium, and calcium in view of its availability and cost.

(Composition)

The composition of the present invention is suitably selected depending on the intended purpose without any restriction, provided that the composition contains the amphiphilic compound.

(Detergent Composition)

The detergent composition of the present invention is suitably selected depending on the intended purpose without any restriction, provided that the detergent contains the amphiphilic compound.

An amount of the detergent composition contained in a detergent is suitably selected depending on the intended purpose without any restriction, but it is preferably 100% by mass to 0.01% by mass.

The detergent composition exhibits sufficient detergency with the activator concentration of 0.01% by mass. For example, the detergent composition can be used as a house cleaning detergent. Moreover, the detergent composition can be used as a soap, such as a bar soap (100% by mass).

The detergent composition may contain one amphiphilic compound independently, or two or more amphiphilic compounds each having the different numbers of the carbon atoms.

A pH of the detergent containing the detergent composition is suitably selected depending on the intended purpose without any restriction, but it is preferably 4 to 12.

Since the amphiphilic compound has an amide bond in its molecular structure, it does not easily cause hydrolysis, which esters often causes, and thus it is stable in a wide pH range.

Accordingly, in the case where the detergent composition is applied for a detergent, it can be widely applied, for example as a detergent for body washing, for a subacidic detergent (having pH of about 5 to about 7) as well as a soap which is alkaline. Moreover, the detergent composition is widely applied, for example, for a relatively strong alkaline detergent such as of pH of about 10, as a detergent for house cleaning.

For the purpose of controlling the pH, a pH regulator may be used in detergent composition.

The pH regulator is not particularly restricted, but it is preferably citric acid, sodium hydroxide, p-toluene sulfonic acid, or the like in view of its availability and cost.

-Other Components-

Other components contained in the detergent containing the detergent composition are suitably selected depending on the intended purpose without any restriction. For example, conventional components formulated into a detergent, such as fragrance materials, other active agents, solvents, and the like can be formulated in accordance with the conventional formulations known in the art.

For example, a builder such as zeolite, sodium(potassium) carbonate, sodium silicate, citric acidsodium, and polyacrylate; a moisturizing agent such as propylene glycol, glycerin, and sorbitol; a viscosity modifier such as methyl cellulose, polyoxyethylene glycol distearate, and ethanol; an antiseptic agent such as methylparaben, and butylparaben; an anti-inflammatory agent such as potassium glycyrrhizinate, and tocopheryl acetate; others such as a sterilizer, a pearling agent, an antioxidant, a fragrance material, a colorant, and an ultraviolet absorber can be formulated, if necessary.

It is also possible that another surfactant is used in combination in the detergent. Preferable examples of such surfactant include: an anionic surfactant such as alkylbenzene sulfonate, $\alpha$-olefin sulfonate, $\alpha$-sulfo fatty acid alkyl ester salts, alkyl sulfuric acid ester salts, polyoxyethylene alkyl sulfuric acid ester salts, alkyl phosphoric acid ester-based surfactants, amino acid-based surfactants, sulfo-succinic acid-based surfactants, taurate-based surfactants, and higher fatty acid salts; and a nonionic surfactant such as alkyl saccharide-based surfactants, and polyoxyethylene alkyl ether-based surfactant.

(Method for Producing Detergent Composition)

The method for producing a detergent composition of the present invention contains heating an alcohol solvent containing $\alpha$-sulfo fatty acid alkyl ester salt, 3-amino-1,2-propanediol, and metal alkoxide to produce the detergent composition. The detergent composition can also be produced by using a solvent other than alcohol, but according to the method for producing the detergent composition of the present invention, which uses the alcohol solvent, use of solvents such as benzene that has carcinogenicity, and toluene that is toxic, can be avoided and thus a production process having low loads to the environment can be realized.

The α-sulfo fatty acid alkyl ester salt is a compound expressed by the following general formula (II):

**R-CH(SO₃A)COOR'**          General Formula (II)

In the general formula (II), R is a C8-16 linear or branched alkyl chain; R' is a C1-4 linear or branched alkyl chain; and A is an alkali metal, an alkaline earth metal, or C6 or smaller protonated amine which may contain a hydroxy group.

The α-sulfo fatty acid alkyl ester salt is appropriately selected depending on a number of carbon atoms of an amphiphilic compound to be produced, without any restriction.

For example, in the case where a 2,3-dihydroxypropyl amide compound of the α-sulfo lauric acid is produced, an α-sulfo lauric acid alkyl ester salt can be used; in the case where a 2,3-dihydroxypropyl amide compound of the α-sulfo myristic acid is produced, an α-sulfo myristic acid alkyl ester salt can be used; in the case where a 2,3-dihydroxypropyl amide compound of the α-sulfo palmitic acid is produced, an α-sulfo palmitic acid alkyl ester salt can be used; and in the case where a 2,3-dihydroxypropyl amide compound of the α-sulfo stearic acid is produced, α-sulfo stearic acid alkyl ester salt can be used.

An amount of the 3-amino-1,2-propanediol to be formulated is not particularly restricted, but it is preferably 0.5 Eq to 1.5 Eq relative to 1 Eq of the α-sulfo fatty acid alkyl ester salt.

When the amount thereof is less than 0.5 Eq, a large amount of the α-sulfo fatty acid alkyl ester salt is remained without being reacted, requiring a complicated purification process. When the amount thereof is more than 1.5 Eq, the cost increases by the excess amount of the alkanolamine, and therefore it is not preferable.

The alcohol solvent is not particularly restricted, and examples thereof include alcohols such as methanol, ethanol, propanol, and butanol.

An amount of the alcohol solvent for use is not particularly restricted, but it is preferably 1 part by mass to 10 parts by mass relative to 1 part by mass of the α-sulfo fatty acid alkyl ester salt.

When the amount thereof is less than 1 part by mass, a reactant is not dissolved, which makes stirring difficult. When the amount thereof is more than 10 parts by mass, a concentration of the resulting reaction product is low, and the productivity thereof is poor, and thus it is not preferable.

The temperature for heating the alcohol solvent containing the α-sulfo fatty acid alkyl ester salt, the 3-amino-1,2-propanediol, and the metal alkoxide is not particularly restricted, but it is preferably 60°C to 170°C.

When the temperature for heating is lower than 60°C, the reaction speed is slow, which causes reduction in the production efficiency. When the temperature for heating is higher than 170°C, part of the reaction product is decomposed, and tinted, and therefore it is not preferable.

The metal alkoxide is not particularly restricted, but it is preferably selected from metal alkoxides such as methanol, ethanol, propanol, or butanol of lithium, potassium, sodium, or magnesium in view of its availability and reactivity.

An amount of the metal alkoxide for use is not particularly restricted, but it is preferably 0.03 Eq to 2 Eq relative to 1 Eq of the α-sulfo fatty acid alkyl ester salt.

When the amount thereof is less than 0.03 Eq, the reaction speed is very slow, which causes reduction in the production efficiency. When the amount thereof is more than 2 Eq, the cost increases, and thus it is not preferable.


Examples

The present invention will be more specifically explained with reference to Examples and Comparative Examples hereinafter, but these Examples shall not be construed as limiting the scope of the present invention in any way.


(Synthesis Example 1)

<Synthesis of sodium methyl α-sulfolaurate (C₁₂MES)>

In a 1L-four necked flask equipped with a thermometer, a stirrer, a dropping funnel, a calcium chloride tube for drying, 54 g (0.25 mol) of methyl laurate, and 540 g of carbon tetrachloride were charged, and to this 24 g (0.3 mol) of sulfuric anhydride was added dropwise while maintaining the reaction temperature at 15°C or lower. After the completion of the instillation of the sulfuric anhydride, the mixture was stirred under the reflux condition for 3 hours.

Next, the reaction solvent was removed by means of an evaporator with a water bath of 50°C, and then 500 mL of methanol was added thereto, followed by stirring for 20 minutes under the reflux condition. Thereafter, the resulting reaction fluid was adjusted to have pH of 7 with a 20% sodium hydroxide solution. The reaction solvent was then removed under the reduced pressure. As foaming occurred during this procedure, isopropanol was added thereto and water was removed while boiling the azeotrope.

The residue (crude product) was heated to 50°C to 60°C, and dissolved in a mixed solvent of ethanol and water (ethanol/water = 9/1(V/V)), and from the resultant insoluble matter was removed by filtration. The resulting filtrate was cooled to 5°C to recrystallize, and precipitates were removed by filtration, followed by subjected to vacuum drying to thereby attain 58 g (yield: 73%) of sodium methyl $\alpha$-sulfolaurate.

The $^1$HNMR ($D_2O$, 50°C) measurement result of the attained synthesized product was: $\delta$0.99 (t, 3H), $\delta$1.41 (br, 16H), $\delta$2.16 (br, 2H), and $\delta$3.90-3.95 (s(3H)+t(1H), 4H), so that the synthesized product was determined as sodium methyl $\alpha$-sulfolaurate.

(Synthesis Example 2)

<Synthesist of sodium methyl $\alpha$-sulfomyristate ($C_{14}$MES)>

In the same manner as in the synthesis of the sodium methyl $\alpha$-sulfolaurate, 71.5 g (yield: 83%) of sodium methyl $\alpha$-sulfomyristate was attained, provided that in the synthesis of $\alpha$-sulfo lauric acid methyl ester ($C_{12}$MES), 54 g (0.25 mol) of metyl laurate was replaced with 61 g (0.25 mol) of methyl myristate.

The $^1$HNMR ($D_2O$, 50°C) measurement result of the obtained synthesized product was: $\delta$0.99 (t, 3H), $\delta$1.40 (br, 20H), $\delta$2.16 (br, 2H), and $\delta$3.90-3.95 (s(3H)+t(1H), 4H) so that the synthesized product was determined as sodium methyl $\alpha$-sulfomyristate ($C_{14}$MES).

(Synthesis Example 3)

<Synthesis of sodium methyl $\alpha$-sulfopalmitate ($C_{16}$MES)>

In the same manner as in the synthesis of the sodium methyl $\alpha$-sulfolaurate, 74.5 g (yield: 80%) of sodium methyl $\alpha$-sulfopalmitate was attained, provided that in the synthesis of $\alpha$-sulfo lauric acid methyl ester ($C_{12}$MES), 54 g (0.25 mol) of metyl laurate was replaced with 68 g (0.25 mol) of methyl palmitate.

The $^1$HNMR ($D_2O$, 50°C) measurement result of the obtained synthesized product was: $\delta$0.99 (t, 3H), $\delta$1.40 (br, 24H), $\delta$2.15 (br, 2H), and $\delta$3.90-3.95 (s(3H)+t(1H), 4H) so that the synthesized product was determined as sodium methyl $\alpha$-sulfopalmitate ($C_{16}$MES).

(Synthesis Example 4)

<Synthesis of sodium methyl $\alpha$-sulfostearate ($C_{18}$MES)>

In the same manner as in the synthesis of the sodium methyl $\alpha$-sulfolaurate, 75 g (yield: 75%) of sodium methyl $\alpha$-sulfostearate was attained provided that in the synthesis of the $\alpha$-sulfo lauric acid methyl ester ($C_{12}$MES), 54 g (0.25 mol) of metyl laurate was replaced with 75 g (0.25 mol) of methyl stearate, and instead of cooling the filtrate to 5°C to recrystalize for recrystalizing the crude product to which the insoluble matter had been removed, adding isopropanol to the filtarate in an amount of 2 times the amount of the water contained in the filtrate and recrystalizing at room temperature to thereby attain 75 g (yield: 75%) of sodium methyl $\alpha$-sulfostearate.

The $^1$HNMR ($D_2O$, 50°C) measurement result of the obtained synthesized product was: $\delta$0.99 (t, 3H), $\delta$1.41 (br, 28H), $\delta$2.15 (br, 2H), $\delta$3.90-3.95 (s(3H)+t(1H), 4H) so that the synthesized product was determined as sodium methyl $\alpha$-sulfostearate ($C_{18}$MES).

<Synthesis of $C_{12}$SF amide alcohol compound, $C_{12}$MES, and diamide compound of $\alpha$-sulfo lauric acid: Examples 1, and 2, and Comparative Examples 1 to 3>

(Example 1)

-Synthesis of $\alpha$-sulfolauric acid monoethanol amide sodium salt-

In a 300 mL-one-necked recovery flask equipped with the Dean-Stark apparatus and a stirring bar, 20 g (63.2 mmol) of $C_{12}$MES obtained in Synthesis Example 1 and 150 mL of toluene were charged, and the mixture was subjected to reflux for 1 hour to remove water.

Thereafter, the resulting reaction solution was cooled to room temperature, and the Dean-Stark apparatus was removed from the flask, and the Dimroth condenser was attached thereto. To the reaction solution, 4.05 g (66.4 mmol) of 2-aminoethanol and 342 mg (6.3 mmol) of sodium methoxide were added, and the mixture was stirred for 4 hours at 90°C.

Thereafter, the resultant was stood to cool to room temperature, and 1.3 mL of 5N hydrochloric acid was added thereto. From the resulting mixture, the solvent was completely removed by azeotropy with isopropanol, to thereby attain a solid crude product.

The residue (the crude product) was heated and dissolved in a methanol/water system at 50°C to 60°C, and recrystallized at -20°C. Then, the precipitates were separated by filtration. The resulting residue was again heated at 50°C to 60°C and dissolved in the metanol/water system, and recrystallized at -20°C. The precipitates were separated by filtration, and subjected to vacuum drying to thereby attain 15.44 g (yield: 70.7%) of $\alpha$-sulfolauric acid monoethanol amide sodium salt.

The $^1$HNMR (D$_2$O, 40°C) measurement result of the obtained synthesized product was: $\delta$0.73 (br, 3H), $\delta$1.15 (br, 16H), $\delta$1.88 (br, 2H), $\delta$.24 (m, 1H), $\delta$3.33 (m, 1H), and 3.58 (br, 3H) so that the synthesized product was determined as $\alpha$-sulfolauric acid monoethanol amide sodium salt.

(Example 2)

-Synthesis of $\alpha$-sulfolauric acid isopropanolamide sodium salt-

In the same manner as the generation of the solid crude product in the synthesis of the $\alpha$-sulfolauric acid monoethanolamide sodium salt, a solid crude product was attained, provided that 4.05 g (66.4 mmol) of 2-aminoethanol was replaced with 4.98 g (66.4 mmol) of 3-amino-2-propanol.

The crude product (residue) was then extracted in a chloroform/methanol/water system, and the water layer was separated therefrom, and the solvent was removed under the reduced pressure by azeotropy with isopropanol, followed by vacuum drying the resultant to thereby attain 18.9 g (yield: 83.2%) of $\alpha$-sulfolauric acid isopropanolamide sodium salt.

The $^1$HNMR (D$_2$O, 25°C) measurement result of the obtained synthesized product is: $\delta$0.71 (br, 3H), $\delta$1.05-1.15 (m+br, 19H), $\delta$1.87 (br, 2H), $\delta$3.18 (br, 2H), 3.60 (br, 1H), and $\delta$3.82 (br, 1H) so that the synthesized product was determined as $\alpha$-sulfolauric acid isopropanolamide sodium salt.

(Comparative Example 1)

-Synthesis of C$_{12}$MES-

C$_{12}$MES was synthesized in the same manner as in Synthesis Example 1.

(Comparative Example 2)

-Synthesis of $\alpha$-sulfolauric acid ethylenediamide sodium salt-

In a 300 mL-one-necked recovery flask equipped with the Dean-Stark apparatus and a stirring bar, 25 g (79 mmol) of C$_{12}$MES obtained in Synthesis Example 1 and 150 mL of toluene were charged, and the mixture was subjected to reflux for 1 hours to remove water. Thereafter, the resulting reaction mixture was cooled to room temperature, and the Dean-Stark apparatus was removed from the flask, and the Dimroth condenser was attached thereto. To the reaction solution, 2.37 g (39.5 mmol) of 1,2-diaminoethane and 4.27g (79 mmoL) of sodium methoxide were added, and the mixture was stirred for 6 hours at 90°C (reaction process).

Thereafter, the resultant was stood to cool to room temperature, and 1.58 mL of 5N hydrochloric acid was added thereto. From the resulting mixture, the solvent was removed under the reduced pressure by azeotropy with isopropanol, to thereby attain a solid crude product.

The residue was heated and dissolved in a methanol/water solution (methanol/water = 85/15(V/V)) at 50°C to 60°C, and the resulting insoluble matter was removed by filtration. From the filterate, the solvent was removed under the reduced pressure by azeotropy with isopropanol. The residue was extracted in a chloroform/methanol/water system, the water layer was separated therefrom, and the solvent was removed under the reduced pressure by azeotropy with isopropanol.

The dry residue was heated and dissolved in an isopropanol/water system at 50°C to 60°C, then recrystallized at room temperature, and the precipitates were separated by filtration, followed by subjected to vacuum drying to thereby attain 12.8 g (yield: 51.8%) of $\alpha$-sulfolauric acid ethylenediamide sodium salt.

The $^1$HNMR (D$_2$O, 25°C) measurement result of the obtained synthesized product was: $\delta$0.75 (br, 6H), $\delta$1.19 (br, 32H), $\delta$1.93 (br, 4H), $\delta$3.31 (br, 4H), and $\delta$3.65 (br, 2H) so that the synthesized product was determined as $\alpha$-sulfolauric acid ethylenediamide sodium salt.

(Comparative Example 3)

-Synthesis of propylene diamide sodium salt of α-sulfolauric acid -

A reaction process was carried out in the same manner as in the reaction process of the ethylene diamide sodium salt of $C_{12}MES$, provided that 2.37 g (39.5 mmol) of 1,2-diaminoethane was replaced with 2.93 g (39.5 mmol) of 1,3-diaminoethane.
The reaction solution was stood to cool to room temperature, and to this 15.8 mL of 5N hydrochloric acid was added, followed by completely removing the solvent by azeotropy with isopropanol.
The residue was heated and dissolved in a methanol/water solution (methanol/water = 9/1(V/V)) at 50°C to 60°C, and the resulting insoluble matter was removed by filtration. From the filterate, the solvent was removed under the reduced pressure by azeotropy with isopropanol. The residue was extracted in a chloroform/methanol/water system, the water layer was separated therefrom, and the solvent was removed under the reduced pressure by azeotropy with isopropanol. The residue was heated and dissolved in an ethanol/water system at 50°C to 60°C, then recrystallized at -20°C, and the precipitates were separated by filtration, followed by subjected to vacuum drying to thereby attain 16.6 g (yield: 58.7%) of α-sulfolauric acid propylenediamide sodium salt.
The $^1HNMR$ ($D_2O$, 25°C) measurement result of the obtained synthesized product was: δ0.72 (br, 6H), δ1.06 (br, 32H), δ1.67 (br, 2H), δ1.87 (br, 4H), δ3.18 (br, 4H), and δ3.64 (br, 2H) so that the synthesized product was determined as a propylene diamide sodium salt of α-sulfolauric acid.

<Synthesis of $C_{14}SF$ amide alcohol compound, and $C_{14}MES$: Examples 3 and 4, and Comparative Example 4>

(Example 3)

-Synthesis of α-sulfomyristic acid monoethanolamide sodium salt-

A solid crude product was obtained in the same manner as the generation of the crude product in the synthesis of the α-sulfolauric acid monoethanolamide sodium salt, provided that 20 g (63.2 mmol) of $C_{12}MES$ obtained in Synthesis Example 1 was replaced with 21.8 g (63.2 mmol) of $C_{14}MES$ obtained in Synthesis Example 2.
The residue (crude product) was heated and dissolved in an ethanol/water solution system (ethanol/water =9/1(V/V)) at 50°C to 60°C, and the resulting insoluble matter was removed by filtration. The filterate was left to stand at room temperature for recrystallization, and the resulting precipitates were separated by filtration, and vacuum dried to thereby attain 6.4 g (yield: 26.9%) of α-sulfomyristic acid monoethanolamide sodium salt.
The $^1HNMR$ ($D_2O$, 40°C) measurement result of the obtained synthesized product was: δ0.89 (t, 3H), δ1.30 (br, 20H), δ2.03 (br, 2H), δ3.37 (m, 1H), δ3.53 (m, 1H), and δ3.75-3.67 (t, 3H) so that the synthesized product was determined as α-sulfomyristic acid monoethanolamide sodium salt.

(Example 4)

-Synthesis of α-sulfomyristic acid isopropanolamide sodium salt-

A solid crude product was obtained in the same manner as the generation of the crude product of $C_{12}MES$ isopropanol amide in the synthesis of the α-sulfolauric acid isopropanolamide sodium salt, provided that 20 g (63.2 mmol) of $C_{12}MES$ obtained in Synthesis Example 1 was replaced with 21.8 g (63.2 mmol) of $C_{14}MES$ obtained in Synthesis Example 2.
The residue (crude product) was extracted in a chloroform/methanol/water system, the water layer was separated therefrom, and the solvent was removed under the reduced pressure by azeotropy with isopropanol. The resultant was subjected to vacuum drying to thereby attain 20.9 g (yield: 85.4%) of α-sulfomyristic acid isopropanolamide sodium salt.
The $^1HNMR$($D_2O$, 25°C) measurement result of the obtained synthesized product was: δ0.70 (br, 3H), δ1.05-1.17 (m+br, 23H), δ1.84 (br, 2H), δ3.15 (br, 2H), δ3.56 (br, 1H), and 3.80 (br, 1H) so that the synthesized product was determined as α-sulfomyristic acid isopropanolamide sodium salt.

(Comparative Example 4)

-Synthesis of $C_{14}MES$-

$C_{14}MES$ was synthesized in the same manner as in Synthesis Example 2.

<Synthesis of $C_{16}$SF amide alcohol compound, and $C_{16}$MES: Examples 5 to 7, and Comparative Example 5>

(Example 5)

-Synthesis of α-sulfopalmitic acid monoethanolamide sodium salt-

A solid crude product was obtained in the same manner as the generation of the crude product of $C_{12}$MES monoethanol amide in the synthesis of the α-sulfolauric acid monoethanolamide sodium salt, provided that 20 g (63.2 mmol) of $C_{12}$MES obtained in Synthesis Example 1 was replaced with 23.5 g (63.2 mmol) of $C_{16}$MES obtained in Synthesis Example 3.
The residue was heated and dissolved in an ethanol/water solution system (ethanol/water =1/9 (V/V)) at 50°C to 60°C, and the resulting insoluble matter was removed by filtration. The filterate was left to stand at room temperature for recrystallization, and the resulting precipitates were separated by filtration, and vacuum dried to thereby attain 16.6 g (yield: 65.3%) of α-sulfopolmitic acid monoethanolamide sodium salt.
The $^1$HNMR ($D_2O$, 25°C) measurement result of the obtained synthesized product was: δ0.71 (t, 3H), δ1.13 (br, 24H), δ1.84 (br, 2H), 3.28 (m, 2H), and 3.56 (br, 3H) so that the synthesized product was determined to be α-sulfopalmitic acid monoethanolamide sodium salt.

(Example 6)

-Synthesis of α-sulfopalmitic acid isopropanolamide sodium salt-

A solid crude product was obtained in the same manner as the generation of the crude product in the synthesis of the α-sulfolauric acid isopropanolamide sodium salt, provided that 20 g (63.2 mmol) of $C_{12}$MES obtained in Synthesis Example 1 was replaced with 23.5 g (63.2 mmol) of $C_{16}$MES obtained in Synthesis Example 3.
The residue (crude product) was heated and dissolved in an ethanol/methanol mixed solvent at 50°C to 60°C, and the resulting insoluble matter was removed by filtration. The filterate was subjected to recrystallization at -20°C, and the resulting precipitates were separated by filtration, and vacuum dried to thereby attain 12.7 g (yield: 48.5%) of α-sulfopalmitic acid isopropanolamide sodium salt.
The $^1$HNMR ($D_2O$, 25°C) measuring result of the obtained synthesized product was: δ0.72 (br, 3H), δ1.02-1.13 (m+br, 27H), δ1.86 (br, 2H), δ3.18 (br, 2H), δ3.60 (br, 1H), and δ3.83 (br, 1H) so that the synthesized product was determined as a-sulfopalmitic acid isopropanolamide sodium salt.

(Example 7)

-Synthesis of α-sulfopalmitic acid 2-(2-hydroxyethoxy)ethylamide sodium salt-

A solid crude product was obtained in the same manner as the generation of the crude product in the synthesis of the α-sulfolauric acid monoethanolamide sodium salt, provided that 20 g (63.2 mmol) of $C_{12}$MES obtained in Synthesis Example 1 was replaced with 23.5 g (63.2 mmol) of $C_{16}$MES obtained in Synthesis Example 3, 4.05 g (66.4 mmol) of 2-aminoethanol was replaced with 6.64 g (66.4 mmol) of 2-(2-aminoethoxy)ethanol, and the amount of the sodium methoxide was changed from 342 mg (6.3 mmol) to 1 Eq relative to $C_{16}$MES.
The residue (crude product) was washed with ethanol to remove the raw material amine. The residue from this was then heated and dissolved in a methanol/water solution (methanol/water =8/2(V/V)) at 50°C to 60°C, and the resulting insoluble matter was removed by filtration. The solvent was then removed from the filterate, to thereby attain 19.5 g (yield: 69.4%) of α-sulfopalmitic acid 2-(2-hydroxyethoxy)ethylamide sodium salt.
The $^1$HNMR ($D_2O$, 25°C) measurement result of the obtained synthesized product was: δ0.71 (t, 3H), δ1.13 (br, 24H), δ1.84 (br, 2H), and δ3.32-3.58 (m, 9H) so that the synthesized product was determined as α-sulfopalmitic acid 2-(2-hydroxyethoxy)ethylamide sodium salt.

(Comparative Example 5)

-Synthesis of $C_{16}$MES-

$C_{16}$MES was synthesized in the same manner as in Synthesis Example 3.

<Synthesis of $C_{18}$SF amide alcohol compound, and $C_{18}$MES: Example 8 and Comparative Example 6>

(Example 8)

-Synthesis of $\alpha$-sulfostearic acid isopropanolamide sodium salt-

A solid crude product was obtained in the same manner as the generation of the crude product in the synthesis of the $\alpha$-sulfolauric acid isopropanolamide sodium salt, provided that 20 g (63.2 mmol) of $C_{12}$MES obtained in Synthesis Example 1 was replaced with 25.3 g (63.2 mmol) of $C_{18}$MES obtained in Synthesis Example 4.
The residue (crude product) was heated and dissolved in an ethanol/methanol mixed solvent at 50°C to 60°C, and the resulting insoluble matter was removed by filtration. The filterate was subjected to recrystallization at -20°C, and the resulting precipitates were separated by filtration, and vacuum dried to thereby attain 15.6 g (yield: 55.5%) of $\alpha$-sulfostearic acid isopropanolamide sodium salt.
The $^1$HNMR ($D_2O$, 25°C) measurement result of the obtained synthesized product was: $\delta$0.71 (br, 3H), $\delta$1.02-1.14 (m+br, 31H), $\delta$1.86 (br, 2H), $\delta$3.17 (br, 2H), $\delta$3.59 (br, 1H), and $\delta$3.82 (br, 1H) so that the synthesized product was determined as $\alpha$-sulfostearic acid isopropanolamide sodium salt.

(Comparative Example 6)

-Synthesis of $C_{18}$MES-

$C_{18}$MES was synthesized in the same manner as in Synthesis Example 4.

(Measurement of rate of protein denaturation)

The detergent compositions of Examples 1 to 8 and Comparative Examples 1 to 6 were subjected to the measurement of the rate of protein denaturation in the following manner.

-Preparation of fluorescent labeled BSA-

In 10 mL of ion-exchanged water, 1 $\mu$mol of BSA (66 kDa, 66 mg), and 10 mL of N-iodeacetyl-N'-(5-sulfo-1-naphtyl)ethylenediamine (IAEDANS, 5 mg) were dissolved, and the solution was stirred for 1 hour at room temperature under the shading condition. After the completion of the reaction between BSA and IAEDANS, dialysis was performed twice using 10 mM of Tris-HCl buffer solution (pH7.5), and the resultant was provided as fluorescent labeled BSA in this test.

-Measurment-

To 0.01% of the fluorescent labeled BSA (50 mM phosphate buffer, pH7.0), 0.01% of the activating agent was added, and the rate of change in the fluorescence intensity was measured just after the addition. The rate of change in the fluorescence intensity induced by a 0.2% SDS solution was indicated as 100% (comparing with excitation wavelength: 340 nm, fluorescence wavelength: 480 nm).
This fluorescent labeled BSA was added to 50 mM phosphate buffer (pH7.0) to prepare a 0.01% fluorescent labeled BSA liquid.
Moreover, a 0.01% synthesized product aqueous solution was prepared with ion-exchanged water, where the synthesized product was each of Examples 1 to 8 and Comparative Examples 1 to 6.
To the 0.01% fluorescent labeled BSA liquid, an equivalent amount of a 0.01% activating agent aqueous solution was added, the fluorescence intensity was measured by a spectrofluorometer (FP-750, JASCO Corporation) just after the addition. Note that, for the measurement of the fluorescence intensity, the light having a wavelength of 480 nm was used as a subject for measuring, among the fluorescent light emitted when light having a wavelength of 340 nm was incident. Based on the measured fluorescence intensity, the rate of protein denaturation was evaluated in the following matter.
The fluorescence intensity just after adding the equivalent amount of ion-exchanged water to the 0.01% fluorescent labeled BSA liquid was determined as a value (X) at which no protein denaturation occurred. Moreover, the fluorescence intensity just after adding the equivalent amount of a 0.2% sodium dodecylsulfate (SDS) solution, as a sample (reference material), to the 0.01% fluorescent labeled BSA liquid was determined as a value (Y) at which 100% thereof was albumioid degenerated. Assuming that the fluorescence intensity just after adding a 0.01% measuring sample (each of Examples 1 to 8 and Comparative Examples 1 to 6) to the 0.01% fluorescent labeled BSA liquid is a value (Z), the rate of protein denaturation can be calculated from the following equation.

$$\text{Rate of protein denaturation (\%)} = (X\text{-}Z)/(X\text{-}Y) \times 100$$

The rate of protein denaturation of each of Examples 1 to 8 and Comparative Examples 1 to 6 is shown in Table 1.
Table 1

| | | Synthesized product | Rate of albuminoid degeneration (%) |
|---|---|---|---|
| | Ex. 1 | α-sulfolauric acid monoethanolamide sodium salt | 11.0 |
| | Ex. 2 | α-sulfolauric acid isopropanolamide sodium salt | 12.5 |
| | Comp. Ex. 1 | $C_{12}$MES | 25.4 |
| | Comp. Ex. 2 | α-sulfolauric acid ethylenediamide sodium salt | 51.0 |
| | Comp. Ex. 3 | α-sulfolauric acid propanediamide sodium salt | 43.0 |
| | Ex. 3 | α-sulfomyristic acid monoethanolamide sodium salt | 20.7 |
| | Ex. 4 | α-sulfomyristic acid isopropanol amide sodium salt | 22.3 3 |
| | Comp. Ex. 4 | $C_{14}$MES | 30.9 |
| | Ex. 5 | α-sulfopalmitic acid monoethanol amide sodium salt | 27.7 |
| | Ex. 6 | α-sulfopalmitic acid isopropanol amide sodium salt | 39.9 |
| | Ex. 7 | α-sulfopalmitic acid 2-(2-hydroxyethyloxy)ethylamide sodium salt | 23.8 |
| | Comp. Ex. 5 | $C_{16}$MES | 86.5 |
| | Ex. 8 | α-sulfostearic acid isopropanolamide sodium salt | 82.3 |
| | Comp. Ex. 6 | $C_{18}$MES | Impossible to measure (insoluble) |
| | Reference material | 0.2% sodium dodecyl sulfate (SDS) | 100.0 |
| | Reference material | Ion-exchanged water | 0.0 |

As it can be understood from Table 1, comparing between the $C_{12}$SF amide alcohol compound and the $C_{12}$MES, Examples 1 and 2 can reduce the protein denaturation more than Comparative Examples 1 to 3 do.
Moreover, comparing between the $C_{14}$SF amide alcohol compound and the $C_{14}$MES, Examples 3 and 4 can reduce the protein denaturation more than Comparative Example 4 does.
Furthermore, comparing between the $C_{16}$SF amide alcohol compound and the $C_{16}$MES, Examples 5 to 7 can reduce the protein denaturation more than Comparative Example 5 does.
Furthermore, comparing between the $C_{18}$SF amide alcohol compound and the $C_{18}$MES, Example 8 can reduce the protein denaturation more than Comparative Example 6 does.

(Skin Irritation Test)

The detergent compositions of Examples 5 and 6 and Comparative Example 5 were each subjected to the skin irritation test in the following manner.
The synthesized products obtained in Examples 5 and 6, and Comparative Example 5 were each separately added to distilled water to prepare the following test sample liquid.

• Example 5

10% α-sulfopalmitic acid monoethanol amide sodium salt aqueous solution

• Example 6

10% α-sulfopalmitic acid isopropanol amide sodium salt aqueous solution

• Comparative Example 5

10% $C_{16}$MES solution
Each of these test sample liquid was applied to guinea pigs (Hartley, clean, ♀, 9 weeks old, number of guinea pigs used: 4) under the following coating conditions.

• Coating condition

The test sample liquid (50 μL) was applied to the skin area of 2 cm × 2 cm
The application was performed once a day for four consecutive days.
The skin which had been coated was then visually judged on the conditions of erythema and scab, and edema based on the following scores, and the evaluation of the skin irritation was evaluated based on the skin irritation score which was the sum of the scores of the erythema and scab and the score of the edema was.

-Erythema and Scab-

0: No erythema
1: Very slight erythema
2: Clear erythema
3: Moderate to severe erythema
4: Extremely severe erythema, or formation of scab

-Edema-

0: No edema

1: Extremely light edema

2: Light edema

3: Moderate edema

4: Severe edema

The evaluation results of the skin irritation are shown in Table 2 below.

Table 2

| | Sample | Skin irritation score, after 4 days (per guinea pig) |
|---|---|---|
| Ex. 5 | α-sulfopalmitic acid monoethanolamide sodium salt | 0.75 |
| Ex. 6 | α-sulfopalmitic acid isopropanolamide sodium salt | 1.00 |
| Comp. Ex. 5 | $C_{16}$MES | 2.00 |

As it can be understood from Table 2, Examples 5 and 6 can reduce the skin irritation by the larger extent than Comparative Example 5 does.

<Synthesis of α-sulfopalmitic acid monoethanolamide sodium salt using alcohol solvent; Examples 9 to 12 and Comparative Example 7>

(Examples 9 to 12 and Comparative Example 7)

Using a pressure-resistant vessel (manufactured by TOKYO RIKAKIKAI CO., LTD.) having a diameter of 30 mm, and a synthesis scale of 14 mL, in a glass vessel having a diameter of 20 mm, 500 mg of $C_{16}MES$ obtained in Synthesis Example 3, a 28% methanol solution of 2-aminoethanol and sodium methoxide, and an alcohol solvent were charged as presented in Table 3, and the mixture was allowed to react at the reaction temperature presented in Table 3 for 90 minutes, followed by removing the solvent from the reaction product, to thereby synthesize α-sulfopalmitic acid monoethanolamide sodium salt of each of Examples 9 to 12.

Moreover, α-sulfopalmitic acid monoethanolamide sodium salt of Comparative Example 7 was synthesized in the same manner as in Example 9, provided that the alcohol solvent was replaced with a toluene solvent.

Alpha-sulfopalmitic acid monoethanolamide sodium salts of Examples 9 to 12 and Comparative Example 7 were each subjected to the $^1$HNMR ($D_2O$, 50°C) measurement to obtain a reaction rate, which was obtained by a ratio of the integral value of 3H proton of the methyl group that was present at the terminal of the alkyl change at the adjacent to 0.99 ppm to the intergral value of proton originated from $-CONH-CH_2-$ adjacent to 3.4 ppm-3.7 ppm. The results are shown in Table 3.

Table 3

|  | Ex.9 | Ex.10 | Ex.11 | Ex.12 | Comp. Ex.7 |
|---|---|---|---|---|---|
| 2-amino ethanol | 1.05 Eq | 1.05 Eq | 1.05 Eq | 1.05 Eq | 1.05 Eq |
| Sodium methoxide | 0.3 Eq | 0.3 Eq | 0.3 Eq | 1 Eq | 0.3 Eq |
| Methanol solvent (mL) | 5 | 5 | - | 5 | - |
| Ethanol solvent (mL) | - | - | 3 | - | - |
| Toluene solvent (mL) | - | - | - | - | 5 |
| Reaction temperature (°C) | 120 | 150 | 130 | 90 | 90 |
| Reactivity (%) | 90 | 80 | 87 | 92 | 95 |
| Color tone | White | Slightly brown | White | White | White |

As explained above, the SF alkanol amide compound of the present invention can be produced using the alcohol solvent without using a toluene solvent that is toxic.

Formulation examples (Example 13 to 18) of a bathroom cleaning detergent, a dish washing detergent, a laundry washing liquid detergent, a hair detergent, and body soap each using the detergent composition of the present invention will be presented hereinafter. Note that, "part" or "parts" presented below represents "part by mass" or "parts by mass".

(Example 13: Bathroom Cleaning Detergent)

| | |
|---|---|
| Alpha-sulfolauric acid monoethanol amide sodium salt | 1.5 parts |
| Sodium laurylamino propionate | 0.4 parts |
| Polyoxyethylene lauryl ether (EO=15) | 0.8 parts |
| Coconut fatty acid potassium salt | 0.8 parts |
| Lauryldimethylamine oxide | 0.7 parts |
| Butyl carbitol | 7.0 parts |
| Ethylene diamine tetraacetate | 1.5 parts |
| Citric acid | 1.2 parts |
| Acrylic acid-maleic acid copolymer *1 | 0.1 parts |
| Sodium hydroxide | optimum |
| Purified water | balance |
| pH | 7.7 |

*1: Sokalan® CP5 (product name), BASF Japan

A bathroom cleaning detergent of the formulation above was produced. The appearance of the prepared product was clear, and the product did not give any feel that dryness of hands would occur when it attached to the hands.

(Example 14: Dish Washing Detergent)

| | |
|---|---|
| Alpha-sulfolauric acid monoethanolamide sodium salt | 28.0 parts |
| EO (2 mol) adduct of lauric acid monoethanolamide | 4.0 parts |
| Lauryldimethylamine oxide | 2.5 parts |
| Polyethylene glycol (weight average molecular weight: 1,000) | 3.0 parts |
| Ethanol | 4.0 parts |
| Sodium benzoate | 2.0 parts |
| p-Toluene sulfonic acid | The amount required for neutralization |
| Citric acidsodium | 1.0 part |
| Purified water | balance |
| pH | 6.5 |

A dish washing detergent of the formulation above was produced. The appearance of the prepared product was clear, and the product did not give any feel that dryness of hands would occur when it attached to the hands.

(Example 15: Laundry Washing Detergent)

| | |
|---|---|
| Alpha-sulfomyristic acid isopropanolamide sodium salt | 20.0 parts |
| Ethanol | 7.0 parts |
| Polyethylene glycol (weight average molecular weight: 400) | 8.0 parts |
| Purified water | balance |

A laundry washing liquid detergent of the formulation above was produced. The appearance of the prepared product was clear, and the product did not give any feel that dryness of hands would occur when it attached to the hands.

(Example 16: Hair Detergent)

| | |
|---|---|
| Alpha-sulfolauric acid monoethanolamide sodium salt | 15.0 parts |
| Lauric acid propyl amide carboxy betaine | 5.5 parts |
| Polyoxyethylene (20) hydrogenated castor oil | 2.5 parts |
| Citric acid | The amount required for neutralization |
| Fragrance material | trace |
| Purified water | balance |
| pH | 5.5 |

A hair detergent of the formulation above was produced. The appearance of the prepared product was clear, and the prepared product did not give any irritation to scalps.

(Example 17: Hair Detergent)

| | |
|---|---|
| Alpha-sulfopalmitic acid isopropanolamide sodium salt | 7.5 parts |
| Alpha-sulfopalmitic acid monoethanol amide sodium salt | 7.5 parts |
| Lauric acid propyl amide carboxy betaine | 5.5 parts |
| Polyoxyethylene (20) hydrogenated castor oil | 2.5 parts |
| Citric acid | The amount required for neutralization |

(continued)

| | |
|---|---|
| Fragrance material | trace |
| Purified water | balance |
| pH | 5.5 |

A hair detergent of the formulation above was produced. The appearance of the prepared product was clear, and the prepared product did not give any irritation to scalps.

(Example 18: Body Soap)

| | |
|---|---|
| Alpha-sulfostearic acid isopropanolamide sodium salt | 10 parts |
| Alpha-sulfopalmitic acid monoethanolamide sodium salt | 10 parts |
| Lauric acid propyl amide carboxy betaine | 4 parts |
| Lauric acid monoethanolamide | 2.5 parts |
| Citric acid | The amount required for neutralization |
| Fragrance material | trace |
| Purified water | balance |
| pH | 6.0 |

A body soap of the formulation above was produced. In terms of appearance, the prepared product was clouded in white, and also the product did not irritate skins at all.

[Amphiphilic Compound]

(Synthesis Example 5)

Sodium methyl $\alpha$-sulfolaurate ($C_{12}$MES) was synthesized in the same manner as in Synthesis Example 1.

(Synthesis Example 6)

Sodium methyl $\alpha$-sulfomyristate ($C_{14}$MES) was synthesized in the same manner as in Synthesis Example 2.

(Synthesis Example 7)

Sodium methyl $\alpha$-sulfopalmitate ($C_{16}$MES) was synthesized in the same manner as in Synthesis Example 3.

(Synthesis Example 8)

Sodium methyl $\alpha$-sulfostearate ($C_{18}$MES) was synthesized in the same manner as in Synthesis Example 4.

<Synthesis of $\alpha$-sulfolauric acid 2,3-dihydroxypropylamide compound, and $C_{12}$MES and $\alpha$-sulfolauric acid diamide compound: Example 19, and Comparative Examples 8 to 10>

(Example 19)

-Synthesis of $\alpha$-sulfolauric acid 2,3-dihydroxypropylamide sodium salt-

In a 300 mL-one-necked recovery flask equipped with the Dean-Stark apparatus and a stirring bar, 20 g (63.2 mmol) of $C_{12}$MES obtained in Synthesis Example 1 and 100 mL of toluene were charged, and the mixture was subjected to reflux for 1 hour to remove water.
Thereafter, the resulting reaction solution was cooled to room temperature, and the Dean-Stark apparatus was removed from the flask, and the Dimroth condenser was attached thereto. To the reaction solution, 4.9 g (63.2 mmol) of 3-amino-1,2-propanediol and 342 mg (6.3 mmol) of sodium methoxide were added, and the mixture was stirred for 1 hour at 90°C. Thereafter, the resultant was stood to cool to room temperature, and 1.3 mL of 5N hydrochloric acid was added thereto. From the resulting mixture, the solvent was completely removed by azeotropy with isopropanol, to thereby attain a solid crude product.

The residue (the crude product) was then extracted in a chloroform/methanol/water system, and the water layer was separated therefrom, and the solvent was removed under the reduced pressure by azeotropy with isopropanol, followed by vacuum drying the resultant to thereby attain 19.9 g (yield: 84%) of $\alpha$-sulfolauric acid 2,3-dihydroxypropyl amide sodium salt.

The $^1$HNMR ($D_2O$, 25°C) measurement result of the obtained synthesized product was: 6H in total, namely, $\delta$0.73 (br, 3H), $\delta$1.13 (br, 16H), $\delta$1.86 (br, 2H), $\delta$3.05-3.17 (m) and $\delta$3.29-3.74(m) so that the synthesized product was determined as $\alpha$-sulfolauric acid 2,3-dihydroxypropyl amide sodium salt.

(Comparative Example 8)

-Synthesis of $C_{12}$MES-

$C_{12}$MES was synthesized in the same manner as in Synthesis Example 1.

(Comparative Example 9)

-Synthesis of $\alpha$-sulfolauric acid ethylenediamide sodium salt-

In a 300 mL-one-necked recovery flask equipped with the Dean-Stark apparatus and a stirring bar, 25 g (79 mmol) of $C_{12}$MES obtained in Synthesis Example 1 and 150 mL of toluene were charged, and the mixture was subjected to reflux for 1 hours to remove water. Thereafter, the resulting reaction mixture was cooled to room temperature, and the Dean-Stark apparatus was removed from the flask, and the Dimroth condenser was attached thereto. To the reaction solution, 2.37 g (39.5 mmol) of 1,2-diaminoethane and 4.27g (79 mmoL) of sodium methoxide were added, and the mixture was stirred for 6 hours at 90°C (reaction process).

Thereafter, the resultant was stood to cool to room temperature, and 1.58 mL of 5N hydrochloric acid was added thereto. From the resulting mixture, the solvent was removed under the reduced pressure by azeotropy with isopropanol.

The residue was heated and dissolved in a methanol/water solution (methanol/water = 85/15(V/V)) at 50°C to 60°C, and the resulting insoluble matter was removed by filtration. From the filterate, the solvent was removed under the reduced pressure by azeotropy with isopropanol. The residue was extracted in a chloroform/methanol/water system, the water layer was separated therefrom, and the solvent was removed under the reduced pressure by azeotropy with isopropanol.

The dry residue was heated and dissolved in an isopropanol/water system at 50°C to 60°C, then recrystallized at room temperature, and the precipitates were separated by filtration, followed by subjected to vacuum drying to thereby attain 12.8 g (yield: 51.8%) of $\alpha$-sulfolauric acid ethylenediamine sodium salt.

The $^1$HNMR ($D_2O$, 25°C) measurement results of the obtained synthesized product was: $\delta$0.75 (br, 6H), $\delta$1.19 (br, 32H), $\delta$1.93 (br, 4H), $\delta$3.31 (br, 4H), and $\delta$3.65 (br, 2H) so that the synthesized product was determined as $\alpha$-sulfolauric acid ethylenediamide sodium salt.

(Comparative Example 10)

-Synthesis of $\alpha$-sulfolauric acid propylenediamide sodium salt-

A reaction process was carried out in the same manner as in the reaction process of the ethylene diamide sodium salt of $C_{12}$MES, provided that 2.37 g (39.5 mmol) of 1,2-diaminoethane was replaced with 2.93 g (39.5 mmol) of 1,3-diaminopropane.

The reaction solution was stood to cool to room temperature, and to this 15.8 mL of 5N hydrochloric acid was added, followed by completely removing the solvent by azeotropy with isopropanol.

The residue was heated and dissolved in a methanol/water solution (methanol/water = 9/1(V/V)) at 50°C to 60°C, and the resulting insoluble matter was removed by filtration. From the filterate, the solvent was removed under the reduced pressure by azeotropy with isopropanol. The residue was extracted in a chloroform/methanol/water system, the water layer was separated therefrom, and the solvent was removed under the reduced pressure by azeotropy with isopropanol.

The residue was heated and dissolved in an ethanol/water system at 50°C to 60°C, then recrystallized at -20°C, and the precipitates were separated by filtration, followed by subjected to vacuum drying to thereby attain 16.6 g (yield: 58.7%) of $\alpha$-sulfolauric acid propylenediamide sodium salt.

The $^1$HNMR ($D_2O$, 25°C) measurement result of the obtained synthesized product was: $\delta$0.72 (br, 6H), $\delta$1.06 (br, 32H), $\delta$1.67 (br, 2H), $\delta$1.87 (br, 4H), $\delta$3.18 (br, 4H), and $\delta$3.64 (br, 2H) so that the synthesized product was determined as $\alpha$-

sulfolauric acid propylenediamide sodium salt.

<Synthesis of α-sulfomyristic acid 2,3-dihydroxypropylamide compound and C$_{14}$MES: Example 20 and Comparative Example 11>

(Example 20)

-Synthesis of α-sulfomyristic acid 2,3-dihydroxypropylamide sodium salt-

Alpha-sulfomyristic acid 2,3-dihydroxypropylamide sodium salt was synthesized in the same manner as in the synthesis of the α-sulfolauric acid 2,3-dihydroxypropylamide sodium salt, provided that in the synthesis of C$_{12}$MES 2,3-dihydroxypropylamide sodium salt, the C$_{12}$MES obtained in Synthesis Example 1 was replaced with 21.8 g (63.2 mmol) of C$_{14}$MES obtained in Synthesis Example 2, to thereby obtain 21.0 g (yield: 82.3%) of α-sulfomyristic acid 2,3-dihydroxypropylamide sodium salt.

The $^1$HNMR (D$_2$O, 25°C) measurement result of the obtained synthesized product was: 6H in total, namely, δ0.73 (br, 3H), δ1.13 (br, 20H), δ1.87 (br, 2H), δ3.04-3.17 (m), and δ3.29-3.73 (m), so that the synthesized product was determined as α-sulfomyristic acid 2,3-dihydroxypropylamide sodium salt.

(Comparative Example 11)

-Synthesis of C$_{14}$MES-

C$_{14}$MES was synthesized in the same manner as in Synthesis Example 6.

<Synthesis of α-sulfopalmitic acid 2,3-dihydroxypropylamide compound and C$_{16}$MES: Example 21 and Comparative Example 12>

(Example 21)

-Synthesis of α-sulfopalmitic acid 2,3-dihydroxypropylamide sodium salt-

Into a 500 ml-one-necked recovery flask equipped with the Dean-Stark apparatus and a stirring bar, 50 g (134.2 mmol) of C$_{16}$MES obtained in Synthesis Example 3 and 300 mL of toluene were charged, and the mixture was subjected to reflux for 1 hour to remove water. Thereafter, the resulting reaction solution was cooled to room temperature, and the Dean-Stark apparatus was removed from the flask, and the Dimroth condenser was attached thereto. To the reaction solution, 12.23 g (134.2 mmoL) of 3-amino-1,2-propanediol and 725 mg (13.4 mmoL) of sodium methoxide were added, and the mixture was stirred for 6 hours at 90°C. Thereafter, the resultant was stood to cool to room temperature, and 2.7 mL of 5N hydrochloric acid was added thereto. From the resulting mixture, the solvent was completely removed by azeotropy with isopropanol, to thereby attain a solid crude product.
To the residue (the crude product), 400 mL of a solution of methanol and water (methanol/water=85/15 (V/V)) was added, and the residue was heated and dissolved therein at 50°C to 60°C, followed by being recrystallized at -20°C. Then, the precipitates were separated by filtration. To the resulting residue, 400 mL of a solution of methanol and water (methanol/water=70/30 (V/V)) was again added, and the residue was heated and dissolved therein at 50°C to 60°C, followed by being recrystallized at -20°C. The precipitates were separated by filtration, and subjected to vacuum drying to thereby attain 40.8 g (yield: 68.2%) of α-sulfopalmitic acid 2,3-dihydroxypropylamide sodium salt.
The $^1$HNMR (D$_2$O, 25°C) measuring result of the obtained synthesized product was: 6H in total, namely, δ0.72 (br, 3H), δ1.13 (br, 24H), δ1.86 (br, 2H), δ3.04-3.17 (m), and δ3.29-3.74 (m) so that the synthesized product was determined as α-sulfopalmitic acid 2,3-dihydroxypropylamide sodium salt.

(Comparative Example 12)

-Synthesis of C$_{16}$MES-

C$_{16}$MES was synthesized in the same manner as in Synthesis Example 7.

<Synthesis of α-sulfostearic acid 2,3-dihydroxypropylamide compound and $C_{18}$MES: Example 22 and Comparative Example 13>

(Example 22)

-Synthesis of α-sulfostearic acid 2,3-dihydroxypropylamide sodium salt-

Alpha-sulfostearic acid 2,3-dihydroxypropylamide was obtained in the same manner as in the synthesis of the α-sulfopalmitic acid 2,3-dihydroxypropylamide, provided that 50 g (134.2 mmol) of $C_{16}$MES obtained in Synthesis Example 3 was replaed with 10 g (25 mmol) of $C_{18}$MES obtained in Synthesis Example 4, and that 50 mL of toluene, 2.5 g (27.5 mmol) of 3-amino-1,2-propanediol, 270 mg (5 mmol) of sodium methoxide, and 1 mL of 5N hydrochloric acid were used, to thereby attain 8.3 g (yield: 72.3%) of α-sulfostearic acid 2,3-dihydroxypropylamide sodium salt.
The $^1$HNMR ($D_2O$, 25°C) measurement result of the obtained synthesized product was: 6H in total, namely, δ0.71 (br, 3H), δ1.14 (br, 28H), δ1.86 (br, 2H), δ3.03-3.16 (m) and δ3.30-3.74 (m) so that the synthesized product was determined to be α-sulfostearic acid 2,3-dihydroxypropylamide sodium salt.

(Comparative Example 13)

-Synthesis of $C_{18}$MES-

$C_{18}$MES was synthesized in the same manner as in Synthesis Example 8.

(Measurement of rate of protein denaturation)

The detergent compositions of Examples 19 to 22 and Comparative Examples 8 to 13 were subjected to the measurement of the rate of protein denaturation in the following manner.

-Preparation of fluorescent labeled BSA-

In 10 mL of ion-exchanged water, 1 μmol of BSA (66 kDa, 66 mg), and 10 mL of N-iodeacetyl-N'-(5-sulfo-1-naphtyl)ethylenediamine (IAEDANS, 5 mg) were dissolved, and the solution was stirred for 1 hour at room temperature under the shading condition. After the completion of the reaction between BSA and IAEDANS, dialysis was performed twice using 10 mM of Tris-HCl buffer solution (pH7.5), and the resultant was provided as fluorescent labeled BSA in this test.

-Measurment-

To 0.01% of the fluorescent labeled BSA (50 mM phosphate buffer, pH7.0), 0.01% of the activating agent was added, and the rate of change in the fluorescence intensity was measured just after the addition. The rate of change in the fluorescence intensity induced by a 0.2% SDS solution was indicated as 100% (comparing with excitation wavelength: 340 nm, fluorescence wavelength: 480 nm).
This fluorescent labeled BSA was added to 50 mM phosphate buffer (pH7.0) to prepare a 0.01% fluorescent labeled BSA liquid.
Moreover, a 0.01% synthesized product aqueous solution was prepared with ion-exchanged water, where the synthesized product was each of Examples 19 to 22 and Comparative Examples 8 to 13.
To the 0.01% fluorescent labeled BSA liquid, an equivalent amount of a 0.01% activating agent aqueous solution was added, the fluorescence intensity was measured by a spectrofluorometer (FP-750, JASCO Corporation) just after the addition. Note that, for the measurement of the fluorescence intensity, the light having a wavelength of 480 nm was used as a subject for measuring, among the fluorescent light emitted when light having a wavelength of 340 nm was incident.
Based on the measured fluorescence intensity, the rate of protein denaturation was evaluated in the following matter. The fluorescence intensity just after adding the equivalent amount of ion-exchanged water to the 0.01% fluorescent labeled BSA liquid was determined as a value (X) at which no protein denaturation occurred. Moreover, the fluorescence intensity just after adding the equivalent amount of a 0.2% sodium dodecylsulfate (SDS) solution, as a sample (reference material), to the 0.01% fluorescent labeled BSA liquid was determined as a value (Y) at which 100% thereof was albumioid degenerated. Assuming that the fluorescence intensity just after adding a 0.01% measuring sample (each of Examples 19 to 22 and Comparative Examples 8 to 13) to the 0.01% fluorescent labeled BSA liquid is a value (Z), the rate of protein denaturation can be calculated from the following equation.

$$\text{Rate of protein denaturation (\%)} = (X\text{-}Z)/(X\text{-}Y) \times 100$$

The rate of protein denaturation of each of Examples 19 to 22 and Comparative Examples 8 to 13 is shown in Table 4.

Table 4

| | Sample | Rate of protein denaturation (%) |
|---|---|---|
| Ex. 19 | $\alpha$-sulfolauric acid 2,3-dihydroxypropylamide sodium salt | 11.0 |
| Comp. Ex. 8 | $C_{12}$MES | 25.4 |
| Comp. Ex. 9 | $\alpha$-sulfolauric acid ethylenediamine diamide sodium salt | 51.0 |
| Comp. Ex. 10 | $\alpha$-sulfolauric acid propanediamine diamide sodium salt | 43.0 |
| Ex. 20 | $\alpha$-sulfomyristic acid 2,3-dihydropropylamide sodium salt | 20.3 |
| Comp. Ex. 11 | $C_{14}$MES | 30.9 |
| Ex. 21 | $\alpha$-sulfopalmitic acid 2,3-dihydroxypropylamide sodium salt | 27.5 |
| Comp. Ex. 12 | $C_{16}$MES | 86.5 |
| Ex. 22 | $\alpha$-sulfostearic acid 2,3-dihydroxypropylamide sodium salt | 42.5 |
| Comp. Ex. 13 | $C_{18}$MES | Impossible to measure (insoluble) |
| Reference Material | 0.2% sodium dodecylsulfate (SDS) | 100.0 |
| Reference Material | Ion-exchanged water | 0.0 |

As it can be understood from Table 4, comparing between the $\alpha$-sulfolauric acid 2,3-dihydroxypropylamide compound and the $C_{12}$MES, Example 19 can reduce the rate of protein denaturation more than Comparative Examples 8 to 10 do.

Moreover, comparing between the $\alpha$-sulfomyristic acid 2,3-dihydroxypropylamide compound and the $C_{14}$MES, Example 20 can reduce the rate of protein denaturation more than Comparative Example 11 does.

Furthermore, comparing between the $\alpha$-sulfopalmitic acid 2,3-dihydroxypropylamide compound and the $C_{16}$MES, Example 21 can reduce the rate of protein denaturation more than Comparative Example 12 does.

Furthermore, comparing between the $\alpha$-sulfostearic acid 2,3-dihydroxypropylamide compound and the $C_{18}$MES, Example 22 can reduce the rate of protein denaturation more than Comparative Example 13 does.

(Skin Irritation Test)

The detergent compositions of Example 21 and Comparative Example 12 were each subjected to the skin irritation test in the following manner.

The synthesized products obtained in Example 21 and Comparative Example 12 were each separately added to distilled water to prepare the following test sample liquid.

• Example 21

10% $\alpha$-sulfopalmitic acid 2,3-dihydroxypropylamide sodium salt solution

• Comparative Example 12

10% $C_{16}$MES solution

Each of these test sample liquid was applied to guinea pigs (Hartley, clean, ♀, 9 weeks old, number of guinea pigs used: 4) under the following coating conditions.

• Coating condition

The test sample liquid (50 µL) was applied to the skin area of 2 cm × 2 cm
The application was performed once a day for four consecutive days.

The skin which had been coated was then visually judged on the conditions of erythema and scab, and edema based on the following scores, and the evaluation of the skin irritation was evaluated based on the skin irritation score which was the sum of the scores of the erythema and scab and the score of the edema was.

-Erythema and Scab-

0: No erythema
1: Very slight erythema
2: Clear erythema
3: Moderate to severe erythema
4: Extremely severe erythema, or formation of scab

-Edema-

0: No edema

1: Extremely light edema

2: Light edema

3: Moderate edema

4: Severe edema

The evaluation results of the skin irritation are shown in Table 5 below.

Table 5

| | Sample | Skin irritation score, after 4 days (per guinea pig) |
|---|---|---|
| Ex. 21 | α-sulfopalmitic acid 2,3-dihydroxypropylamide sodium salt | 0.00 |
| Comp. Ex. 12 | $C_{16}$MES | 2.00 |

As it can be understood from Table 5, Example 21 can reduce the skin irritation by larger extent than Comparative Example 12 does.

<Synthesis of α-sulfopalmitic acid 2,3-dihydroxypropylamide sodium salt using alcohol solvent; Example 23>

(Example 23)

Using a pressure-resistant vessel (manufactured by TOKYO RIKAKIKAI CO., LTD.) having a diameter of 30 mm, and a synthesis scale of 14 mL, in a glass vessel having a diameter of 20 mm, 500 mg of $C_{16}$MES obtained in Synthesis Example 3, 128 mg (1.41 mmol) of 3-aminopropanediol, 75 mg (0.4 mmol) of a 28% methanol solution of sodium methoxide, and 5 mL of methanol were charged, and the mixture was allowed to react at 120°C for 90 minutes. After removing the solvent from the reaction solution, 4 mL of a solution of methanol and water (methanol/water=85/15 (V/V)) was added to the residue, and the residue was heated and dissolved therein at 50°C to 60°C, followed by being recrystallized at -20°C. The resulting precipitates were separated by filtration, followed by subjected to vacuum drying to thereby attain 352 mg (yield: 59%) of α-sulfopalmitic acid 2, 3-dihydroxypropylamide sodium salt.
The $^1$HNMR ($D_2O$, 25°C) measurement result of the obtained synthesized product was: 6H in total, namely, δ0.72 (br, 3H), δ1.13 (br, 24H), δ1.86 (br, 2H), δ3.04-3.17 (m) and δ3.29-3.74 (m) so that the synthesized product was determined as α-sulfopalmitic acid 2,3-dihydroxypropylamide sodium salt.

In the manner presented above, the α-sulfofatty acid 2,3-dihydroxypropylamide compound of the present invention can be produced using an alcohol solvent without using a toluene solvent or the like that is toxic.

Formulation examples (Example 24 to 29) of a bathroom cleaning detergent, a dish washing detergent, a laundry washing liquid detergent, a hair detergent, and body soap each using the detergent composition of the present invention will be presented hereinafter. Note that, "part" or "parts" presented below represents "part by mass" or "parts by mass".

(Example 24: Bathroom Cleaning Detergent)

| | |
|---|---|
| Alpha-sulfolauric acid 2,3-dihydroxypropylamide sodium salt | 1.5 parts |
| Sodium laurylamino propionate | 0.4 parts |
| Polyoxyethylene lauryl ether (EO=15) | 0.8 parts |
| Coconut fatty acid potassium salt | 0.8 parts |
| Lauryldimethylamine oxide | 0.7 parts |
| Butyl carbitol | 7.0 parts |
| Ethylene diamine tetraacetate | 1.5 parts |
| Citric acid | 1.2 parts |
| Acrylic acid-maleic acid copolymer *1 | 0.1 parts |
| Sodium hydroxide | optimum |
| Purified water | balance |
| pH | 7.7 |
| *1: Sokalan® CP5 (product name), BASF Japan | |

A bathroom cleaning detergent of the formulation above was produced. The appearance of the prepared product was clear, and the product did not give any feel that dryness of hands would occur when it attached to the hands.

(Example 25: Dish Washing Detergent)

| | |
|---|---|
| Alpha-sulfomyristic acid 2,3-dihydroxypropylamide sodium salt | 28.0 parts |
| EO (2 mol) adduct of lauric acid monoethanolamide | 4.0 parts |
| Lauryldimethylamine oxide | 2.5 parts |
| Polyethylene glycol (weight average molecular weight: 1,000) | 3.0 parts |
| Ethanol | 4.0 parts |
| Sodium benzoate | 2.0 parts |
| p-Toluene sulfonic acid | The amount required for neutralization |
| Citric acidsodium | 1.0 part |
| Purified water | balance |
| pH | 6.5 |

A dish washing detergent of the formulation above was produced. The appearance of the prepared product was clear, and the product did not give any feel that dryness of hands would occur when it attached to the hands.

(Example 26: Laundry Washing Liquid Detergent)

| | |
|---|---|
| Alpha-sulfomyristic acid 2,3-dihydroxypropylamide sodium salt | 4.0 parts |

(continued)

| Alpha-sulfopalmitic acid 2,3-dihydroxypropylamide sodium salt | 16.0 parts |
| Ethanol | 7.0 parts |
| Polyethylene glycol (weight average molecular weight: 400) | 8.0 parts |
| Purified water | balance |

A laundry washing liquid detergent of the formulation above was produced. The appearance of the prepared product was clear, and the product did not give any feel that dryness of hands would occur when it attached to the hands.

(Example 27: Hair Detergent)

| Alpha-sulfolauric acid 2,3-dihydroxypropylamide sodium salt | 15.0 parts |
| Lauric acid propyl amide carboxy betaine | 5.5 parts |
| Polyoxyethylene (20) hydrogenated castor oil | 2.5 parts |
| Citric acid | The amount required for neutralization |
| Fragrance material | trace |
| Purified water | balance |
| pH | 5.5 |

A hair detergent of the formulation above was produced. The appearance of the prepared product was clear, and the prepared product did not give any irritation to scalps.

(Example 28: Hair Detergent)

| Alpha-sulfomyristic acid 2,3-dihydroxypropylamide sodium salt | 7.5 parts |
| Alpha-sulfopalmitic acid 2,3-dihydroxypropylamide sodium salt | 7.5 parts |
| Lauric acid propyl amide carboxy betaine | 5.5 parts |
| Polyoxyethylene (20) hydrogenated castor oil | 2.5 parts |
| Citric acid | The amount required for neutralization |
| Fragrance material | trace |
| Purified water | balance |
| pH | 5.5 |

A hair detergent of the formulation above was produced. The appearance of the prepared product was clear, and the prepared product did not give any irritation to scalps.

(Example 29: Body Soap)

| Alpha-sulfopalmitic acid 2,3-dihydroxypropylamide sodium salt | 10 parts |
| Alpha-sulfostearic acid 2,3-dihydroxypropylamide sodium salt | 10 parts |
| Lauric acid propyl amide carboxy betaine | 4 parts |
| Lauric acid monoethanolamide | 2.5 parts |
| Citric acid | The amount required for neutralization |
| Fragrance material | trace |
| Purified water | balance |
| pH | 6.0 |

A body soap of the formulation above was produced. In terms of appearance, the prepared product was clouded in white, and also the product did not irritate skins at all.

[Industrial Applicability]

The detergent composition of the present invention is suitably applied for a detergent composition for body washing, dish washing, bathroom cleaning, and house cleaning, and particularly suitably used as a detergent such as a bathroom cleaning detergent, a dish washing detergent, a liquid laundry detergent, a hair detergent, and a body soap.

**Claims**

1. A detergent composition, comprising:

    a compound expressed by the following general formula (1):

$$C_{10}\text{-CH (SO}_3A)\text{-CONR'R''} \qquad \text{General Formula (1)}$$

    where $C_{10}$ is a C10 linear or branched alkyl chain; R' is a hydrogen atom or a C1-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group.

2. A detergent composition, comprising:

    a compound expressed by the following general formula (2):

$$C_{12}\text{-CH (SO}_3A)\text{-CONR'R''} \qquad \text{General Formula (2)}$$

    where $C_{12}$ is a C12 linear or branched alkyl chain; R' is a hydrogen atom or a C1-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group.

3. A detergent composition, comprising:

    a compound expressed by the following general formula (3):

$$C_{14}\text{-CH (SO}_3A)\text{-CCNR'R''} \qquad \text{General Formula (3)}$$

    where $C_{14}$ is a C14 linear or branched alkyl chain; R' is a hydrogen atom or a C1-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group,
    wherein the detergent composition is for body washing, dish washing, bathroom cleaning, or house cleaning.

4. A detergent composition, comprising:

    a compound expressed by the following general formula (4):

$$C_{16}\text{-CH (SO}_3A)\text{-CONR'R''} \qquad \text{General Formula (4)}$$

    where $C_{16}$ is a C16 linear or branched alkyl chain; R' is a hydrogen atom or a C-3 alkyl group which may contain a hydroxy group; R'' is a C2-6 alkyl group which contains one hydroxy group; and A is an alkali metal, an alkaline earth metal, or C6 or smaller protonated amine which may contain a hydroxy group,
    wherein the detergent composition is for body washing, dish washing, bathroom cleaning, or house cleaning.

5. A method for producing a detergent composition, comprising:

    heating an alcohol solvent containing an α-sulfo fatty acid alkyl ester salt expressed by the following general formula (5), alkanolamine, and metal alkoxide to produce the detergent composition as defined in any one of claims 1 to 4:

**R-CH(SO$_3$A)COOR'''**          General Formula (5)

where R is a C10, 12, 14, or 16 linear or branched alkyl chain; R''' is a C1-4 linear or branched alkyl chain; and A is an alkali metal, an alkaline earth metal, or C6 or smaller protonated amine which may contain a hydroxy group.

6. An amphiphilic compound, having a molecular structure expressed by the following general formula (I):

**R-CH(SO$_3$A)-CONHCH$_2$CH(OH)CH$_2$OH**          General Formula (I)

where R is a C 8-16 linear or branched alkyl chain; and A is an alkali metal, an alkaline earth metal, or C6 or smaller protonated amine which may contain a hydroxy group.

7. A composition, comprising the amphiphilic compound as defined in claim 6.

8. A detergent composition, comprising the amphiphilic compound as defined in claim 6.

9. A method for producing a detergent composition, comprising:

heating an alcohol solvent containing an α-sulfo fatty acid alkyl ester salt expressed by the following general formula (II), 3-amino-1,2-propanediol, and metal alkoxide to produce the detergent composition as defined in claim 8:

**R-CH(SO$_3$A)COOR'**          General Formula (II)

where R is a C8-16 linear or branched alkyl chain; R' is a C1-4 linear or branched alkyl chain; and A is an alkali metal, an alkaline earth metal, or C6 or smaller protonated amine which may contain a hydroxy group.

**EP 2 380 955 A1**

<table>
<tr><td colspan="2">INTERNATIONAL SEARCH REPORT</td><td>International application No.<br>PCT/JP2009/069554</td></tr>
</table>

A. CLASSIFICATION OF SUBJECT MATTER
*C11D1/28*(2006.01)i, *A61K8/46*(2006.01)i, *A61Q5/02*(2006.01)i, *A61Q19/10*
(2006.01)i, *C07C309/17*(2006.01)i, *C11D11/04*(2006.01)i, *C07B61/00*
(2006.01)n
According to International Patent Classification (IPC) or to both national classification and IPC

B. FIELDS SEARCHED

Minimum documentation searched (classification system followed by classification symbols)
C11D1/28, A61K8/46, A61Q5/02, A61Q19/10, C07C231/02, C07C303/22,
C07C309/17, C11D11/04, C07B61/00

Documentation searched other than minimum documentation to the extent that such documents are included in the fields searched
Jitsuyo Shinan Koho            1922–1996    Jitsuyo Shinan Toroku Koho    1996–2010
Kokai Jitsuyo Shinan Koho      1971–2010    Toroku Jitsuyo Shinan Koho    1994–2010

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | JP 54-15911 A  (Union Generale de Savonnerie),<br>06 February 1979 (06.02.1979),<br>claims<br>& US 4194986 A       & GB 1563182 A<br>& DE 2804324 A1    & FR 2379601 A<br>& BE 863513 A       & CH 619264 A<br>& MX 148382 A      & AR 215044 A<br>& AT 69578 A       & NL 7801081 A<br>& NO 780354 A      & NZ 186371 A<br>& AU 3289678 A    & PT 67593 A<br>& SE 7801138 A    & ES 466579 A<br>& BR 7800599 A    & CA 1093418 A<br>& DK 44878 A       & IL 53939 A<br>& LU 78976 A      & IT 1093806 B<br>& OA 5860 A       & GR 62091 A | 1,2<br>3-9 |

☒ Further documents are listed in the continuation of Box C.　　☐ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search<br>05 February, 2010 (05.02.10) | Date of mailing of the international search report<br>23 February, 2010 (23.02.10) |
|---|---|
| Name and mailing address of the ISA/<br>Japanese Patent Office | Authorized officer |
| Facsimile No. | Telephone No. |

Form PCT/ISA/210 (second sheet) (April 2007)

| | **INTERNATIONAL SEARCH REPORT** | International application No. |
|---|---|---|
| | | PCT/JP2009/069554 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | SMITH, F. D. et al., Soap-based detergent formulations. IX. α-Sulfo fatty alkanolamides as lime soap dispersing agents, Journal of the American Oil Chemists' Society, 1974, 51(10), 435-8 | 1-4<br>5-9 |
| X<br>A | WEIL, J. K. et al., Amides of α-sulfonated palmitic and stearic acids, Journal of the American Oil Chemists' Society, 1960, 37, 295-7 | 1-4<br>5-9 |
| Y | JP 11-508873 A  (Akzo Nobel N.V.),<br>03 August 1999 (03.08.1999),<br>claims; page 9, line 29 to page 10, line 16;<br>page 11, line 26 to page 12, line 3<br>& US 5646318 A        & EP 833814 A1<br>& WO 1996/033967 A1    & DE 69612745 T<br>& BR 9608282 A | 5 |
| Y | JP 7-310092 A  (Kao Corp.),<br>28 November 1995 (28.11.1995),<br>claims; synthesis example 2<br>(Family: none) | 6-9 |
| Y | JP 6-293722 A  (Kao Corp.),<br>21 October 1994 (21.10.1994),<br>claims; paragraphs [0011] to [0013]; example 2<br>(Family: none) | 6-9 |
| A | HACKETT, Walter J. et al., Evaluation of a new tall-oil-derived fatty acid in floor-polish use, Chem. Specialties Mfrs. Assoc., Proc., 46th Ann. Meeting, Dec., 1959, 239-43, (abstract) CA plus [online];Chemical Abstract Service, Columbus, Ohio, USA. [retrieved on 4 February 2010] Retrieved from: STN Tokyo, Japan Association for International Chemical Information CA Plus Accession no. 1960 83703 | 1-9 |

Form PCT/ISA/210 (continuation of second sheet) (April 2007)

## INTERNATIONAL SEARCH REPORT

International application No.

PCT/JP2009/069554

**Box No. II    Observations where certain claims were found unsearchable (Continuation of item 2 of first sheet)**

This international search report has not been established in respect of certain claims under Article 17(2)(a) for the following reasons:

1. ☐ Claims Nos.:
    because they relate to subject matter not required to be searched by this Authority, namely:

2. ☐ Claims Nos.:
    because they relate to parts of the international application that do not comply with the prescribed requirements to such an extent that no meaningful international search can be carried out, specifically:

3. ☐ Claims Nos.:
    because they are dependent claims and are not drafted in accordance with the second and third sentences of Rule 6.4(a).

**Box No. III    Observations where unity of invention is lacking (Continuation of item 3 of first sheet)**

This International Searching Authority found multiple inventions in this international application, as follows:
    Both claims 1 and 6 are independent claims, and the inventions of these claims share a common subject matter "an alkanolamide of an α-sulfo fatty acid".
    However, an alkanolamide of an α-sulfo fatty acid is disclosed in, for example, JP 54-15911 A which is a document cited in the International Search Report, and cannot be regarded as a special technical feature. Consequently, there is no technical relationship involving the same or corresponding special technical feature between the inventions in these claims, and the present application does not comply with the requirement of unity of invention.

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant.   Consequently, this international search report is restricted to the invention first mentioned in the claims; it is covered by claims Nos.:

**Remark on Protest**          ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (April 2007)

**EP 2 380 955 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6330084 A

**Non-patent literature cited in the description**

- *J. Am. Oil Chem. Soc.,* 1960, vol. 37, 295-297
- *J. Am. Oil Chem. Soc.,* 1962, vol. 39, 490-496
- *J. Am. Oil Chem. Soc.,* 1974, vol. 51, 435-438